# EUROPEAN PATENT SPECIFICATION

(11) **EP 1 726 297 B1**
(45) Date of publication and mention of the grant of the patent: **18.08.2010**
(21) Application number: 05011065.9
(22) Date of filing: 23.05.2005
(51) Int. Cl.: A61K 8/63, A61Q 7/02

(54) **Composition comprising wortmannin and its topical use for reducing human hair growth**
Zusammensetzung enthalend Wortmannin und ihre topische Verwendung zur Haarwuchshemmung
Composition comprenant de la wortmannine et son utilisation pour inhiber la croissance capillaire

(43) Date of publication of application: 29.11.2006
(73) Proprietor: Reckitt Benckiser (UK) Limited, Slough, Berkshire SL1 3UH (GB)
(72) Inventor: de la Torre, Frederic, Dansom Lane Hull HU8 7DS (GB); Tobin, Desmond John, BD16 2EH West Yorkshire (GB)
(74) Representative: Bowers, Craig Malcolm

(56) References cited:
- EP-A- 0 610 519
- WO-A-01/07021
- WO-A-90/12577
- WO-A-98/02134
- US-A- 5 554 608
- US-A1- 2001 033 849

## Description

The invention relates to a method for reducing hair growth in humans, particularly for cosmetic purposes.

In humans, particularly women, visible body hair is considered unattractive in some cultures. Many and various procedures have been employed to remove unwanted hair, including shaving, electrolysis, application of depilatory creams or lotions which dissolve hair, waxing, plucking (manual or mechanical using an epilator), laser therapy and therapeutic antiandrogens, sometimes by injection. These procedures generally have drawbacks associated with them. Shaving may cause skin abrasion or cuts, and can lead a perception of roughness caused by sharp, stubbly hairs as the hair reappears after shaving. Electrolysis can be particularly effective at keeping a treated area free of hair for prolonged periods of time, but can be time consuming and painful. Electrolysis also carries the potential risk of scarring if excessive currents are applied.

Depilatory creams are effective, but the regime of their application must be carefully controlled to avoid risk of skin irritation. Waxing and plucking of hairs is an intrinsically painful and uncomfortable process. Also, for waxing to be effective, the hairs must be about 5 mm in length, which means that the skin may look hairy before rewaxing will be effective. Antiandrogens, which maybe used to treat female hirsutism, may have unwanted side effects because of their effect on the hormone balance in the body.

Hence it would be desirable to provide a chemically or biochemically based method for the inhibition, reduction or delay of hair growth in humans.

It has previously been disclosed that the rate and character of hair growth can be altered by applying to the skin inhibitors of certain enzymes. These inhibitors include inhibitors of 5-alpha reductase, ornithine decarboxylase, S-adenosylmethionine decarboxylase, gamma- glutamyl transpeptidase, and transglutaminase. These chemicals function by modification of a biochemical pathway to inhibit the production of a final product, and so potentially lead to unwanted side effects in the user arising from the loss of specific amino acids.

EP0996409 discloses serine proteases to induce programmed cell death and apoptosis in the follicular papillae to affect changes in mammalian hair growth.

It is desirable to provide alternate methods for the inhibition, reduction or prevention of hair growth in mammals which induce cell death by apoptosis and which have minimal effect on biochemical amino acid pathways. It is also desirable to use chemical compounds which are derived from natural sources, in that such chemicals are more likely to be accepted by users as less harmful than synthetic materials. It is also desirable to combine anti-inflammatory effects on the skin along with hair growth inhibition, delay or reduction. This is because the various hair removal processes described above may cause or exacerbate skin inflammation.

Wortmannin (11-(acetyloxy)-1,6b,7,8,9a,10,11,11b-octahydro-1-(methoxymethyl)-9a,11b-di-methyl-[1S-(1α, 6bα,9aβ,11α,11bβ)]-3H-furo[4,3,2-de]indeno[4,5-h]-2-benzopyran-3,6,9-trione) is a fungal metabolite capable of entering intact cells and can induce cell death by apoptosis.

In one aspect, the invention provides a non-therapeutic cosmetic method of reducing human hair growth comprising selecting an area of skin from which reduced growth is desired and applying to the area of skin a cosmetically or dermatologically acceptable composition comprising wortmannin in an amount effective to reduce hair growth. The unwanted hair growth may be normal but undesirable from a cosmetic perspective or may result, for example, from the symptoms of a disease or an abnormal condition such as hirsutism.

In order to put the method of the invention into practice, the wortmannin is suitably included in a topical composition along with a dermatologically or cosmetically acceptable vehicle or carrier. Suitably the vehicle or carrier is adapted to be spread upon the skin. Accordingly, the present invention also relates to topical compositions comprising a dermatologically and/or cosmetically acceptable carrier and wortmannin in an amount effective to reduce hair growth.

Examples of suitable vehicles or carriers include acetone, alcohols, creams, lotions or gels. When the carrier is a cream or lotion, it is preferably in the form of an oil in water or a water in oil emulsion.

The composition may be a solid, semi-solid, or liquid. The composition may be a cosmetic or a therapeutic product in the form of an ointment, lotion, foam, cream, gel, or solution. The composition may also be in the form of a shaving preparation or an after-shave product intended for application to the skin after shaving.

The wortmannin is suitably present in the composition at a level from 0.001% to 30% percent by weight of the composition, preferably 0.01% to 10%, more preferably from 0.3% to 6%.

In addition, the present invention relates to the use of wortmannin in the preparation of a medicament for reducing hair growth. In another aspect, the invention provides a cosmetic method of reducing unwanted human hair growth by applying to the skin wortmannin in an amount effective to reduce hair growth.

In addition, the composition may include one or more other types of hair growth reducing agents.

The concentration of the wortmannin in the composition may be varied over a wide range; the reduction of hair growth increases as the amount of wortmannin applied increases per unit area of skin. The maximum amount effectively applied is limited only by the rate at which the wortmannin penetrates the skin. The effective amounts may range, for example, from 10 ng/cm² to 1000 µg/cm², preferably from 100 ng/cm² to 100 µg/cm² even more preferably 3 µg/cm² to 60 µg/cm².

The vehicle or carrier for the wortmannin can be inert or can possess cosmetic, physiological and/or pharmaceutical benefits of its own. Vehicles can be formulated with liquid or solid emollients, solvents, thickeners, humectants and/or powders. Emollients include cetyl alcohol, stearyl alcohol, triglyceride oils, oleyl alcohol, isopropyl laurate, polyethylene glycols, petroleum jelly, and esters such as myristyl myristate. Solvents include ethyl alcohol, isopropanol, acetone, diethylene glycol, ethylene glycol, propylene glycol, butylene glycol, hexylene glycol, 2-methyl-1,3-propanediol, dimethyl isosorbide, dimethyl sulfoxide, and dimethyl formamide.

The composition also can include components that enhance the penetration of the inhibitor into the skin and/or to the site of action. Examples of penetration enhancers include urea, cis-fatty acids (e.g., oleic acid, palmitoleic acid), acetone, laurocapram, dimethylsulfoxide, 2-pyrrolidone, oleyl alcohol, glyceryl-3 stearate, propan-2-ol, myristic acid isopropyl ester, cholesterol, and propylene glycol.

A penetration enhancer can be added suitably at concentrations of 0.1% to 20% or preferably 0.5% to 5% by weight. The composition also can be formulated to provide a reservoir within or on the surface of the skin to provide for a continual slow release of the wortmannin. The composition also may be formulated to evaporate slowly from the skin while the wortmannin slowly penetrates into the surface of the skin.

The composition should be topically applied to a selected area of the body from which it is described to reduce hair growth. For example, the composition can be applied to the face, particularly to the beard area of the face, i.e., the cheek, neck, upper lip, and chin. The composition also may be used as an adjunct to other methods of hair removal including shaving, waxing, mechanical epilation, chemical depilation, electrolysis and or laser-assisted hair removal.

The composition can also be applied to the legs, groin region (bikini area), arms, torso or armpits. The composition is particularly suitable for reducing the growth of unwanted hair in women having hirsutism or other conditions. In humans, the composition should be suitably applied once a day, preferably at least twice a day, to achieve a perceivable reduction in hair growth. Perception of reduced hair growth could occur as early as 24 hours or 48 hours (for instance, between normal shaving intervals) following use, but may require several months. Reduction in hair growth is demonstrated when, for example, the rate of hair growth is slowed, the need for removal is reduced, the subject perceives less hair on the treated site, or quantitatively, when the weight of hair removed is reduced.

In alternative embodiments, the topically active therapeutic or cosmetic composition may be optionally combined with other ingredients such as moisturisers, foaming agents, cosmetic adjuvants, anti-oxidants, surfactants, foaming agents; conditioners, humectants, fragrances, viscosity modifiers, buffering agents, preservatives, and the like, in order to produce cosmetic or pharmaceutical products such as shaving creams, shaving gels, shaving powders, chemical depilatory creams and the like.

Another embodiment of the present invention, is the use of the composition of the invention for delaying human hair growth in combination with a short-term or instantaneous method of hair removal. By "in combination with" it is meant that the application of the composition of the invention to the surface of the skin within one hour or less of the hair removal by the other method.

The short-term or instantaneous method of hair removal can be performed by any method well known in the art, including, but not limited to chemical or mechanical depilation such as, shaving, wax depilation, chemical depilation, mechanical plucking with an epilator and combinations thereof. By short-term or instantaneous it is meant that the hair removal method removes the hair by cutting, dissolution or plucking, but does not prevent or inhibit regrowth of the hair. Chemical depilation means the dissolution of hair by a depilation agent such as an alkaline thioglycolate.

The time at which the topically active or cosmetic composition is applied to the skin, as well as the amount of time for which the composition remains on the skin, may vary, but is suitably within one hour of instantaneous hair removal. Preferably the composition is applied to the skin surface either immediately before, immediately after or simultaneously with instantaneous hair removal. More preferably, the topically active pharmaceutical or cosmetic composition is applied either simultaneously with or immediately following hair removal, most preferably immediately following instantaneous hair removal, and is left on the skin for a period sufficient to obtain an effect to delay hair growth, preferably at least five minutes and more preferably at least fifteen minutes. Most preferably, the composition is allowed to remain on the skin indefinitely to permit absorption into the skin. An advantage of this aspect of the invention is that the wortmannin combines an anti-inflammatory effect with the inhibition, delay or reduction in hair growth.

In another aspect, the invention provides a method for the preparation of a composition for reducing human hair growth that comprises incorporating wortmannin in a hair-growth inhibiting concentration into a cosmetically and/or dermatologically acceptable carrier.

The invention will be further demonstrated by reference to the following examples:

### Examples

A study was carried out to assess the effects of wortmannin on cell proliferation for dermal fibroblasts, follicular dermal papilla fibroblasts derived from 5 normal adults (3 females and 2 males).

A study carried out to assess the effects of wortmannin on cell proliferation for hair follicle keratinocytes was performed on cells from 1 normal healthy individual (female).

A study was carried out to assess the effects of wortmannin on growth of Anagen (IV) intact hair follicles isolated intact from 2 females aged 49 and 60.

A composition was prepared comprising wortmannin at 10 milli-molar in a vehicle of dimethyl sulfoxide (DMSO). The levels of wortmannin were such that when the composition was added to the growth medium in the experiments, the level of wortmannin in the experimental medium was 50, 70, 100 or 140 micro-molar and the respective level of DMSO was 0.5%, 0.7%, 1.0%, 1.4% by weight.

The control experiments were carried out using as the growth medium CM (control medium only as detailed below) and CV (control medium containing the DMSO vehicle but free of wortmannin). The experiments using the wortmannin composition were carried out using the wortmannin compositions in the control medium.

For experiments with dermal fibroblasts and follicular dermal papilla fibroblasts, Control Medium was RPMI 1640 media supplemented with penicillin/streptomycin, L-glutamine and Foetal Calf Serum.

For experiments with hair follicle keratinocytes, Control Medium was keratinocyte serum-free medium (KSF-M) medium supplemented with penicillin/streptomycin, L-glutamine, epidermal growth factor and bovine pituitary extract.

For experiments with intact hair follicle, Control Medium was Williams E medium supplemented with fungizone, penicilin/streptomycin, L-glutamine, insulin-like growth factor and hydrocortisone.

Results in terms of percentage cell number change after 8 days of the cells/follicles in the compositions were as follows in table 1.

| | Control Medium CM | Control Medium +Vehicle CV | 50 µm | 70 µm | 100 µm | 140 µm |
|---|---|---|---|---|---|---|
| Dermal fibroblasts (normalized on CV results) | | 100 | | 25 | | |
| dermal papilla fibroblasts (normalized on CV results) | | 100 | | 34 | | |
| Hair follicle keratinocytes (normalized on CV results) | | 100 | 75 | 40 | 30 | |
| Whole hair follicles (length increase in %) | 26 | 9 | | 4 | | 4 |

Hence it can be seen that the wortmannin inhibits hair follicle cell related growth and hair follicle length growth (fiber elongation) at the concentrations used.

## Claims

1. A non-therapeutic cosmetic method for reducing human hair growth comprising selecting an area of skin from which reduced hair growth is desired and applying to the area of skin a cosmetically acceptable composition comprising wortmannin in an amount effective to reduce hair growth.

2. The method of claim 1 wherein the level of wortmannin in the composition is from 0.001 to 10 percent by weight of the composition.

3. The method of any preceding claim, wherein the wortmannin is applied to the skin in an amount of from 0.01 to 100 micrograms of wortmannin per square centimetre of skin.

4. The method of any preceding claim, wherein the area of skin is selected from areas on the face of a human, on a leg of the human, on an arm of the human, in an armpit of the human, in the groin area of the human, on the torso of the human.

5. The method of any preceding claim wherein the composition is applied to the area of skin in combination with mechanical or chemical depilation.

6. The use of wortmannin in the preparation of a medicament for reducing human hair growth.

7. A composition for reducing human hair growth comprising from 0.3 to 6 percent by weight of wortmannin in a cosmetically and/or dermatologically acceptable carrier.

## Patentansprüche

1. Nichttherapeutisches kosmetisches Verfahren zur Verringerung des Haarwuchses bei einem Menschen, umfassend ein Auswählen eines Hautbereichs, an dem der verringerte Haarwuchs gewünscht wird, und Auftragen auf den Hautbereich einer kosmetisch unbedenklichen Zusammensetzung, die Wortmannin in einer Menge umfasst, die wirksam ist, um den Haarwuchs zu verringern.

2. Verfahren nach Anspruch 1, wobei der Anteil an Wortmannin in der Zusammensetzung 0,001 bis 10 Gewichtsprozent der Zusammensetzung beträgt.

3. Verfahren nach einem der vorhergehenden Ansprüche, wobei das Wortmannin in einer Menge von 0,01 bis 100 Mikrogramm Wortmannin pro Quadratzentimeter Haut auf die Haut aufgetragen wird.

4. Verfahren nach einem der vorhergehenden Ansprüche, wobei der Hautbereich aus Bereichen im Gesicht eines Menschen, auf einem Bein des Menschen, auf einem Arm des Menschen, in einer Achselhöhle des Menschen, in der Leistengegend des Menschen und auf dem Rumpf des Menschen ausgewählt wird.

5. Verfahren nach einem der vorhergehenden Ansprüche, wobei die Zusammensetzung in Kombination mit mechanischer oder chemischer Enthaarung auf den Hautbereich aufgetragen wird.

6. Verwendung von Wortmannin bei der Herstellung eines Arzneimittels zum Verringern des Haarwuchses bei einem Menschen.

7. Zusammensetzung zum Verringern des Haarwuchses bei einem Menschen, umfassend 0,3 bis 6 Gewichtsprozent Wortmannin in einem kosmetisch und/oder dermatologisch unbedenklichen Träger.

## Revendications

1. Procédé cosmétique non thérapeutique destiné à réduire la pousse des poils d'une personne, comprenant la sélection d'une zone de la peau dont on souhaite réduire la pousse des poils et l'application sur la zone de la peau d'une composition acceptable sur le plan cosmétique comprenant de la wortmannine dans une quantité efficace pour réduire la pousse des poils.

2. Procédé selon la revendication 1, dans lequel la concentration de la wortmannine dans la composition est de 0,001 à 10 pour cent en poids de la composition.

3. Procédé selon l'une quelconque des revendications précédentes, dans lequel la wortmannine est appliquée sur la peau dans une quantité de 0,01 à 100 microgrammes de wortmannine par centimètre carré de peau.

4. Procédé selon l'une quelconque des revendications précédentes, dans lequel la zone de la peau est choisie parmi les zones situées sur le visage d'une personne, sur une jambe de la personne, sur un bras de la personne, au niveau d'une aisselle de la personne, au niveau de l'aine de la personne ou sur le torse de la personne.

5. Procédé selon l'une quelconque des revendications précédentes, dans lequel la composition est appliquée sur la zone de la peau en association avec une épilation mécanique ou chimique.

6. Utilisation de wortmannine dans la préparation d'un médicament destiné à réduire la pousse des poils d'une personne.

7. Composition destinée à réduire la pousse des poils d'une personne, comprenant de 0,3 à 6 pour cent en poids de wortmannine dans un excipient acceptable sur le plan cosmétique et/ou dermatologique.
